# EUROPEAN PATENT APPLICATION

(11) **EP 4 778 449 A1**
(43) Date of publication of application: **22.07.2026**
(21) Application number: 25796643.2
(22) Date of filing: 15.01.2025
(51) Int. Cl.: A61B 5/00

(54) **PERIPHERAL NERVE STIMULATION CONTROL SYSTEM AND METHOD**

(30) Priority: 13.11.2024 KR 20240160702; 20.12.2024 KR 20240192221
(71) Applicant: POSTECH Research and Business Development Foundation, Pohang-si, Gyeongsangbuk-do 37673 (KR)
(72) Inventor: PARK, Sung Min, Pohang-si, Gyeongsangbuk-do 37666 (KR); LIM, Young Soo, Pohang-si, Gyeongsangbuk-do 37673 (KR)
(74) Representative: Nederlandsch Octrooibureau
(86) International application number: PCT/KR2025/099011
(87) International publication number: WO 2026/106444

(57) **Abstract**

A peripheral nerve stimulation control system, the system comprising: an implantable device configured to electrically stimulate a peripheral nerve and measure evoked compound action potential (ECAP) from the peripheral nerve; a controller configured to receive feedback of the evoked compound action potential (ECAP) measured after stimulation of the peripheral nerve and determine a threshold value of the evoked compound action potential (ECAP); and a stimulation determiner configured to determine a stimulation intensity for stimulating the peripheral nerve according to the threshold value is provided.

## Description

### [Technical Field]

The present disclosure relates to a peripheral nerve stimulation control system and method, and more particularly, to a peripheral nerve stimulation control system and method capable of measuring a therapeutic effect and an appropriate intensity in a peripheral nerve system through a compound action potential-based feedback control system.

### [Background Art]

Neuromodulation is a clinical approach that inhibits or excites a target nerve to modulate abnormal neural activity through artificial stimulation using electrical, magnetic, or ultrasound energy according to established clinical protocols. To date, various neuromodulation treatments, including deep brain stimulation (DBS) for Parkinson's disease, transcranial magnetic stimulation (TMS) for mental illness, and epidural spinal cord stimulation (SC) for chronic pain management, have been approved by the FDA and have proven statistically significant patient results in modern clinical settings.

Traditionally, neuromodulation techniques have been applied mainly to the central nervous system, resulting in lower accessibility. However, there is a growing interest in peripheral nerve regulation, which provides higher accessibility and serves as an important pathway for nerve signals that directly regulate various organs. As a result, studies related to peripheral nerve stimulation (PNS) for chronic diseases such as high blood pressure and bladder dysfunction have been actively conducted. The ongoing study aims to address various medical needs and improve patient outcomes by expanding the clinical application of PNS. This development indicates that further improvement of PNS may lead to more accurate and effective treatment solutions, which may provide significant potential for customized treatment.

The current PNS protocol is determined periodically by a medical professional who analyzes the patient's condition and adjusts treatment regimens by modifying factors such as duration, intensity, and frequency of stimulation. However, the optimal treatment protocol may be constantly changing due to individual physiological conditions, drug interactions, and changes in the disease progression stage. In addition, fluctuations in the electrode-neural interface can have a significant impact on physiological equilibrium, potentially interfering with obtaining meaningful results from applied stimuli. Despite standardized protocol management across patient groups, achieving consistent results for all individuals remains a challenge. This variability is evident in certain studies, and this study reports differences in effect between equal groups. There are also documented cases in which excessive electrical stimulation is applied to some individuals in the control group, putting tissue and nerve damage at risk. Despite these risks, methods to utilize the maximum stimulation intensity at the patient's tolerance level are still being implemented. There are cases where the resulting inefficiency or side effect is greater than the advantage of neuromodulation. This instability has led the development of control methods that continuously integrate relevant physiological feedback without relying on empirical observation or periodic coordination based on experience.

The technological environment of neuromodulation is rapidly evolving on the back of the development of a bioelectronic system that enables real-time signal recording and closed-loop control. Capturing a comprehensive physiological signal can provide quantitative analysis to improve the precision of treatment intervention. In particular, neural signal monitoring has made significant advances in DBS and SCS. The use of local field potential (LFP) and spike assays for closed-loop control 39 greatly improved the management of diseases mediated by central nervous system disorders. These established interventions, however, have advanced in treating diseases such as epilepsy and even hypertension, but LFP and spike assays have not been effectively delivered to the peripheral nervous system, making adaptation and application difficult. To address the limitations of current PNS, attention should be shifted to a control system that utilizes measurable and effective functions within the peripheral nervous system to maximize the results of neuromodulation.

Among these PNS treatments, percutaneous tibial nerve stimulation (PTNS) is recognized as having the potential to provide high scalability across various PNS treatments. This potential is not limited to specific physiological parameters of chronic diseases, but rather depends on nerve signal feedback, particularly the evoked compound action potential (ECAP) observed throughout the nerve strands. Accordingly, development of a customized device for measurement and stimulation is essential because the device is designed to be fully implantable for transcutaneous tibial nerve stimulation (PTNS), but a system for this has not yet been developed. overactive bladder (OAB) refers to a disease that is commonly accompanied by daytime urination and night urination, with urinary urgency (urgency urination, strong and sudden desire to urinate) in the absence of other clear causes such as urinary tract infections. PNS has been studied as a non-pharmacological intervention treatment for adults of overactive bladder syndrome, but the above-described tibial nerve stimulation system for controlling bladder activity and an electrode for the same have not been developed.

### [Disclosure]

### [Technical Problem]

Accordingly, an object of the present invention is to provide a tibial nerve stimulation system in which measurement and stimulation for PNS treatment are integrated.

### [Technical Solution]

According to an aspect of the present invention, there is provided a peripheral nerve stimulation control system, the system comprising: an implantable device configured to electrically stimulate a peripheral nerve and measure evoked compound action potential (ECAP) from the peripheral nerve; a controller configured to receive feedback of the evoked compound action potential (ECAP) measured after stimulation of the peripheral nerve and determine a threshold value of the evoked compound action potential (ECAP); and a stimulation determiner configured to determine a stimulation intensity for stimulating the peripheral nerve according to the threshold value.

In one example of the present invention, the peripheral nerve stimulation system is implantable in vivo.

In one example of the present invention, the peripheral nerve stimulation control system further comprises a wireless charging unit configured to wirelessly supply power to the device.

In one example of the present invention, the controller integrates the measured evoked compound action potential (ECAP) value.

In one example of the present invention, the control unit determines a point at which the second derivative of the integrated evoked compound action potential (ECAP) value exceeds twice the previous result value and shows a significant increase as the threshold value.

In one example of the present invention, the peripheral nerve is a percutaneous tibial nerve.

The present invention also provides a peripheral nerve stimulation control system for overactive bladder (OAB) treatment, comprising the peripheral nerve stimulation system mentioned above.

The present invention also provides a peripheral nerve stimulation control method, the method comprising: electrically stimulating a peripheral nerve; measuring an evoked compound action potential (ECAP) from the peripheral nerve; receiving feedback of the measured evoked compound action potential (ECAP) and determining a threshold value of the evoked compound action potential (ECAP); and determining a stimulation intensity for stimulating the peripheral nerve according to the threshold value.

In one example of the present invention, the determining of the threshold value of the evoked compound action potential (ECAP) comprises integrating the measured evoked compound action potential (ECAP), and the threshold value is a point at which a second derivative of the integrated evoked compound action potential (ECAP) value exceeds twice a previous result value and shows a significant increase.

In one example of the present invention, the peripheral nerve is a percutaneous tibial nerve.

In one example of the present invention, the determined stimulation strength is an electrical stimulation strength obtained by stimulating the peripheral nerve at a point where the threshold value is determined.

### [Advantageous Effects]

The present invention can optimize stimulation parameters by using ECAP as quantitative nerve feedback, unlike the prior art having the problem that it is not certain that the corresponding electrical stimulation leads to further a desired neuromodulation effect in motor reflex.

### [Brief Description of Drawings]

FIG. 1 is a schematic diagram of an ECA-based stimulation intensity regulation mechanism for P kinetic NS stimulation.
FIG. 2 is a schematic diagram of a customized design for application of the TNS of the present system and shows a result of comparing the urination suppression effect between the control method by the kinetic reaction of the TNS and the control method of the present system.
FIG. 3 illustrates an experimental protocol timeline for a TNS experiment.
FIG. 4 is a result of threshold testing experiment progress for threshold value analysis.

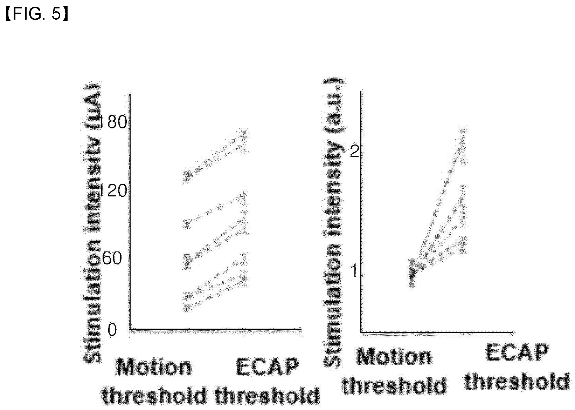

FIG. 5 is a comprehensive analysis result of a threshold value of motor reaction and a threshold value of ECAP.
FIG. 6 is the result of analysis of changes in the urination pattern after stimulation using each threshold value.
FIG. 7 is a comprehensive analysis result of the difference in urination interval length after stimulation using each threshold value.
FIG. 8 is an experimental design for system testing after implantation in an autonomic rat.
FIG. 9 is a diagram illustrating an experimental protocol timeline for experimenting with an autonomic rat.
FIG. 10 is a diagram illustrating a threshold value analysis process performed in a rat performing an autonomous action.
FIG. 11 is a flowchart illustrating ECAP-based control method according to an embodiment of the present disclosure.

### [Best Mode]

Hereinafter, a preferred embodiment of the present invention will be described in detail with reference to the accompanying drawings.

Before describing the present invention in detail, the terms or words used in the present specification should not be interpreted as being unconditionally limited to typical or dictionary meanings, and the inventor of the present invention may appropriately define and use the concepts of various terms in order to describe his or her invention in the best way.

Furthermore, it should be noted that these terms or words should be interpreted as meanings and concepts consistent with the technical idea of the present invention.

That is, the terms used in the present specification are only used to describe the preferred embodiments of the present invention, and are not intended to specifically limit the contents of the present invention.

It should be noted that these terms are defined in consideration of various possibilities of the present invention.

In addition, in the present specification, a singular expression may include a plural expression unless the context clearly indicates a different meaning.

In addition, it should be noted that even if it is similarly expressed in plural, it may include a singular meaning.

In the case of describing that a component "includes" another component throughout the specification, it may mean that any other component may be further included, rather than excluding any other component, unless otherwise described.

Furthermore, when a component is described as being "present inside" or connected to and installed in another component, the component may be directly connected to or in contact with the other component.

In addition, the components may be installed to be spaced apart from each other at a predetermined distance, and when the components are installed to be spaced apart from each other at a predetermined distance, a third component or means for fixing or connecting the corresponding component to another component may exist.

Meanwhile, it should be noted that the description of the third components or means may be omitted.

On the other hand, when a component is described as being "directly connected" or "directly connected" to another component, it should be understood that there is no third component or means.

Likewise, other expressions describing a relationship between components, that is, "between" and "directly between" or "adjacent to" and "directly adjacent to" should be interpreted as having the same purpose.

In addition, in the present specification, terms such as "one surface", "the other surface", "one side", "the other side", "first", "second", and the like are used to clearly distinguish one component from other components.

However, it should be noted that the meaning of the corresponding element is not limitedly used by such terms.

In addition, it should be understood that terms related to positions such as "up", "down", "left", "right", and the like in the present specification, if used, refer to relative positions in the corresponding drawings with respect to corresponding components.

In addition, unless absolute positions are specified for these positions, it should not be understood that these position-related terms refer to absolute positions.

Furthermore, in the specification of the present invention, terms such as "unit", "system", "module", "device", if used, mean a unit capable of processing one or more functions or operations.

It should be noted that this may be implemented in hardware or software, or a combination of hardware and software.

In the drawings attached to the present specification, the size, position, coupling relationship, and the like of each component constituting the present invention may be partially exaggerated, reduced, or omitted in order to sufficiently and clearly convey the spirit of the present invention or for convenience of description, and thus the proportion or scale may not be exact.

In addition, in the following description of the present invention, a detailed description of a configuration determined to unnecessarily obscure the gist of the present invention, for example, a known technology including the prior art may be omitted.

To solve the above problems, the present invention provides a closed-loop control system that monitors from stimulation to evaluate neural activation and thus optimizes stimulation parameters,
and further selects tibial nerve stimulation (TNS) as an application of this closed-loop neural regulation method to evaluate the in vivo performance and validity of the system according to the present invention and uses ECAP quantitative neural feedback to optimize stimulation parameters.

The systems and methods according to the invention are highly accurate and adaptable to the direct neural interface and can provide high scalability over a variety of PNS treatments, since the system according to the invention is not limited to specific physiological parameters of chronic disease but is dependent on neural signal feedback, in particular ECAP observed over the neural strand.

The present invention provides an integrated device designed to be fully implantable and customized for ECAP measurement and peripheral nerve stimulation and provides feedback by monitoring ECAP from the electrodes to nerve activation evaluation so that the control system can adjust stimulation parameters accordingly.

To evaluate the in vivo performance and feasibility of the integrated system according to an embodiment of the present invention, tibial nerve stimulation (TNS) was selected as an application program of the closed-loop neural regulation method, but the scope of the present invention is not limited thereto.

TNS to which a system according to an embodiment of the present invention is applied is a new treatment for alleviating overactive bladder (OAB) symptoms, and the effect of the system according to the present invention was evaluated by monitoring the adjusted bladder activity.

Hereinafter, the present invention will be described in more detail with reference to preferred examples.

### Embodiments

### Device firmware

The system according to the present invention operates with a CC1350 microcontroller unit (Texas Instruments) that controls the RHS2116 electrophysiology interface (Intan Technologies). The firmware was written in the Code Composer Studio (v12, Texas Instruments) and controlled through the LabView GUI (National Instruments Corporation). A development kit (Texas Instruments Inc. ) was used to establish a wireless connection for the PC control operation of the device. On the host PC, the GUI allowed control of device functions and adjustment of stimulation parameters, displayed measurement results, and enabled data transfer for further analysis.

### Circuit development

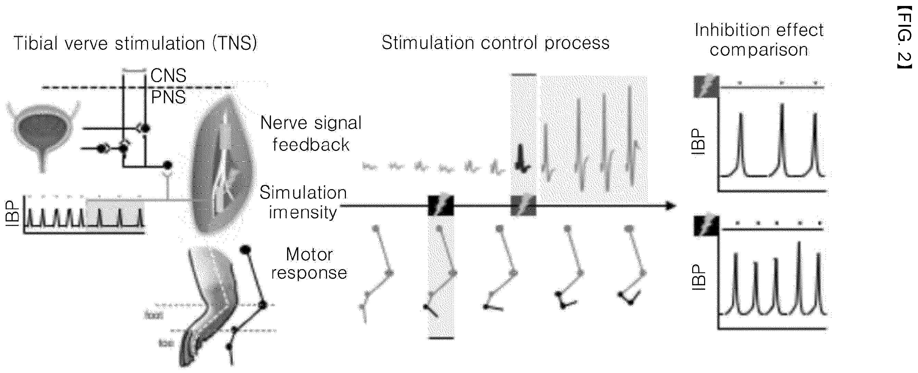

The system according to the invention was designed to withstand implantation conditions and was powered by a 3.7V Li-Po battery with a WPT charging option. A 3.3V low dropout regulator (ADP151, Analog Devices Inc.) has been incorporated to ensure proper operation of the main chip. In addition, a ±5V buck-boost converter (LT3582, Analog Devices Inc.) was used to generate a two-phase signal required for stimulation. An 433MHz antenna (ANT1204, pulse electronics) has been integrated for wireless communication. A functional diagram of the circuit design is shown in supplemental FIG. 2. PCB size was 15mm×18mm.

### Electrode implantation

In the present invention, the measurement electrode implanted in vivo is a mesh-type gold electrode coated with a polymer such as GeIMA. The electrode was located in the sciatic nerve to measure a nerve signal, and in particular, a response signal to stimulation applied from the tibial nerve was measured, and a provoking complex potential signal was extracted. On the other hand, a stimulation electrode for providing electrical stimulation to the peripheral nerve is located at the tibial nerve, and in an embodiment of the present invention, tibial nerve stimulation is performed using a nerve cuff electrode manufactured by a microprobe capable of bio-transplantation.

### Wireless power transfer design

The peripheral nerve stimulation system according to the present invention is charged by a wireless charging method, and the wireless power unit (system) for this is designed as an WPT Rx coil dedicated for devices. The WPT Tx coil was integrated into a cylindrical cage, and both coils were tuned to a frequency of 6.78MHz. The system according to the present invention incorporates a linear voltage regulator (XCM414, Torex Semiconductor Ltd.) to enable WPT charging of the battery. The WPT Rx coil was designed to surround the edge of the PCB to optimize space utilization with a size of 17mm×20mm. The WPT Tx coil was connected to a wireless power amplifier (EPC9512, Efficient Power Conversion Corp.) and a DC power supply for WPT charging. (PWS4305, Tektronix Inc.)

### Packaging

This package was composed of ceramic and cobar, which are biocompatible materials, and brazed together to facilitate welding of the implanted conditions. This design prioritizes non-metallic (ceramic) parts covering 80% or more of the case surface area to improve wireless connection efficiency. The case lid is also made of Kovar material with SA271082 Rev3 (Morgan's high-end material) for electrode connection. The case was laser welded after assembly for hermetically sealing. The dimensions of the packaging case are 20mm × 25mm × 8 mm including the lid.

### Rat preparation

All procedures were approved by the Pohang University of Science and Technology's Animal Care Use Committee (POSTECH IACUC). A Sprague-Dawley rat (270-300g body weight, 8 weeks old, male) was used in the study. The rat received short-term anesthesia with isoflurane (Pyramal Critical Care, Inc.) for a surgical procedure (isoflurane 2%, flow rate 0.5-0.6L/min, 30-40 min). Postoperative anesthesia was converted to urethane (Sigma-Aldrich) through intraperitoneal injection (urethane saline at 20%, body weight 1.0-1.2g/kg) to maintain an autonomous system function for urination reflection.

The sciatic nerve was measured by placing a cuff electrode developed in a customized manner by approaching the outside of the thigh. Similarly, the tibial nerve was accessed through the ankle and stimulated by applying a nerve cuff electrode (NC-1-2-250SS-1-2-Sut-20SS, microprobe for life sciences). The rat bladder was exposed and the catheter was connected to a pressure transducer (BP-100, iWorx System Inc.) and a syringe pump (pump 11 Elite I/W Dual, v3.0.6, Harvard Device) that controls IBP to insert the catheter to control IBP. DAQ module (IX-RA-834, iWorx System Inc.) and Labscribe (v3, iWorx System Inc.) were used for physiological measurements.

Additional surgical procedures were required for free-moving rat. The catheter was expanded and connected to neck button (Instech Laboratories Inc.) to minimize motion interference and fix the catheter connection from the body to the external environment. The system according to the present invention was implanted in the subcutaneous lumbar region and maintained connection with the electrode. Surgical incisions were sutured and disinfected before the rat recovered from isoflurane anesthesia.

### Analysis

### ECAP threshold value analysis and stimulation control

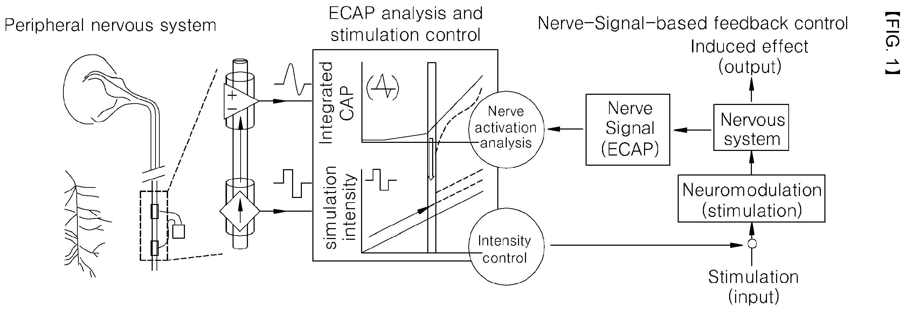

FIG. 1 is an ECA-based stimulation intensity adjustment mechanism for PNS stimulation, and FIG. 2 is a design schematic diagram for TNS application of the present invention, and shows the result of comparing the effect of inhibiting urination between the control method by the kinetic response of TNS and the control method of the present system.

Referring to FIGS. 1 and 2, the measurement results transmitted from the apparatus of FIG. 1 were analyzed to capture an ECAP response for evaluating an ECA threshold value. The ECAP signal was integrated to quantify neural activation, and the ECAP threshold indicates that the second derivative of the integrated ECAP signal was determined to be a point that exceeded twice the previous resulting value, resulting in a significant increase.

The ECAP threshold value (i.e., the electrical stimulation intensity of the stimulation electrode at the time when the threshold value comes out) was selected as the stimulation intensity, and the nerve signal was continuously monitored to maintain the ECAP signal level throughout the stimulation. The protocol is as illustrated in FIG. 2, and in the present invention, the stimulation intensity becomes the electrical stimulation intensity obtained by stimulating the peripheral nerve at the point where the threshold value is determined.

### System evaluation through a TNS application

In the clinical setting, TNS treatment is commonly used to relieve symptoms of urinary tract disorders such as urinary incontinence and OAB. TNS aims to reduce urinary muscle hyperactivity and relieve the urgency and frequency symptoms of the bladder. Although the exact mechanism of TNS is still uncertain, it is believed that this effect may occur due to the modulation of the centripetal signal caused by bladder swelling to maintain the persistence of the bladder. Quantitative analysis of the increased ablation intervals achieved by the applied stimuli by monitoring bladder activity via measurements of intrabladder pressure (IBP) was performed according to FIGS. 1 to 2.

The bioelectronic systems according to the present invention are specially designed for TNS with implantable devices and electrode configurations that are tailored to meet the requirements of TNS treatment. The system evaluates neural activity by delivering electrical stimulation to the tibial nerves while measuring response signals from the sciatic nerves.

Using the ECAP analysis described in FIGS. 1-2, the system adjusts the stimulation intensity to ensure accurate control and effective neural regulation. The conventional TNS uses toe tremors as a criterion for determining stimulation intensity, but the system according to the present invention analyzes nerve activation increase as a selection criterion using ECAP as a feedback function. In order to evaluate the performance of the feedback-based stimulation optimization system according to the present disclosure, the motor response (foot tremors) was monitored through electromyogram to be compared with the existing method.

### Threshold evaluation for stimulation intensity control

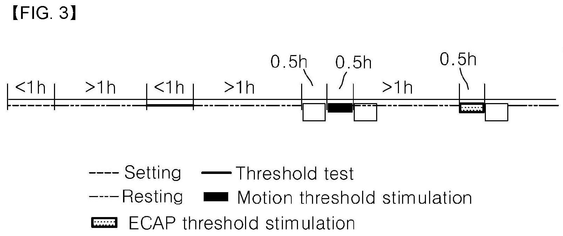

The experimental protocol shown in FIG. 3 is a result of summarizing the threshold value test steps performed to evaluate the motion threshold value and the ECAP threshold value of each subject. During this test, the intensity of stimulation gradually increased (step 5 µA at 20 to 255 µA within a safe range for direct neural stimulation), and the neural signals and electromyographic responses were continuously measured.

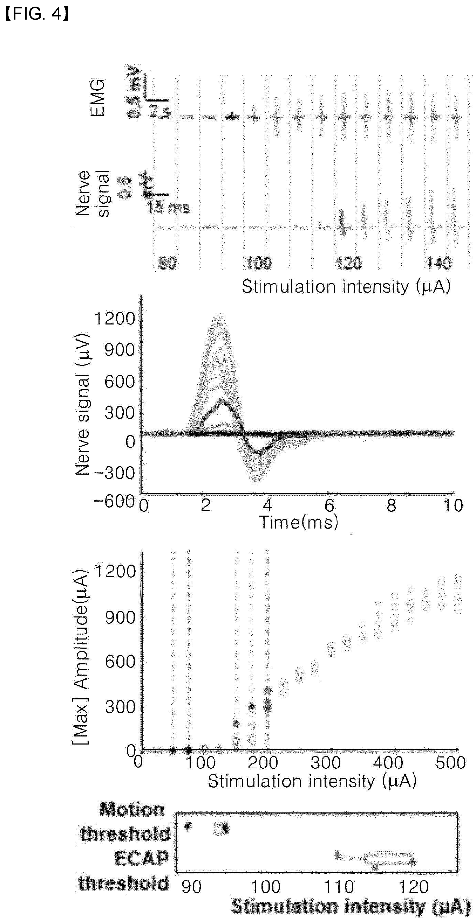

FIG. 4 is a result of presenting a threshold value analysis result of representative data obtained during the test, and the upper figure shows EMG and neural signal responses to the applied stimulation intensity.

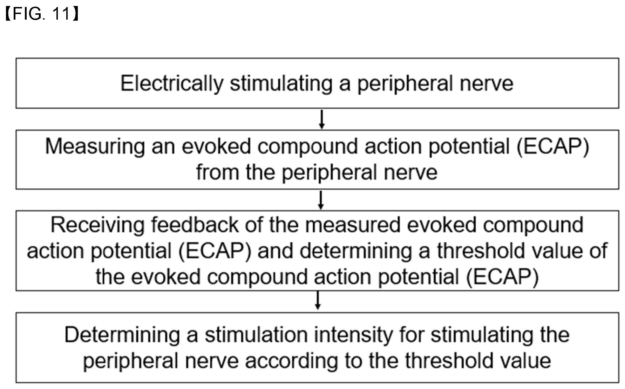

In FIG. 4, the motion threshold was defined as the intensity of stimulation at which EMG activation occurred, and the ECAP threshold was determined by a significant increase in the amplitude of ECAP as analyzed by the rate of change of the integrated ECAP. The threshold test ensured accurate measurement and confirmation of the two thresholds with an average of 94±2.2 µA for the motion threshold and an average of 117±4.5 µA for the ECAP threshold. FIG. 11 shows the motion threshold and ECAP threshold measured in eight mice, with the motion threshold consistently being lower than the ECAP threshold.

The tibial nerve branching from the sciatic nerve is composed of a motor nerve and a sensory nerve that encompass various types of nerve fibers. These fibers have distinct physical and electrophysiological properties. As fiber diameter decreases and myelination decreases, activating and propagating the activity potential requires progressively higher stimulation strength in the following order: Aα-, Aβ-, A δ, C fibers. The afferent nerves of the urinary bladder mainly contain A δ and C fibers, and the motor response occurs in Aα fibers. A higher ECAP threshold relative to the motor threshold suggests that additional nerve fibers were activated as the ECAP threshold was reached. This approach enables more precise intensity adjustment using ECAP as a specific physiological marker and provides better quantitative control than conventional methods.

### TNS effect comparison for system evaluation

Reducing the yaw frequency for OAB treatment is one of the important factors. In the main experiment, the effect of increasing the urination interval between the group stimulated at the kinetic threshold and the ECAP threshold intensity was compared.

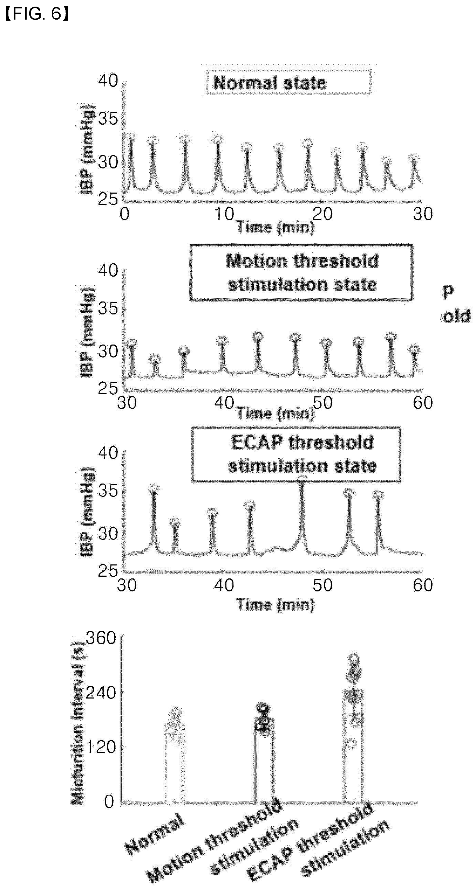

FIG. 6 is a result showing a representative urination pattern measured at this step. Exercise threshold stimulation was performed first, followed by ECAP threshold stimulation, and a minimum 1-hour rest period was placed between the tests to minimize residual effects. IBP was monitored throughout the experiment to assess urination activity and compare the results of the stimulation test to steady-state. The lower figure shows the results of the urination interval analysis. Kinetic threshold stimulation slightly increased the urination interval, while ECAP threshold stimulation increased 38% compared to the normal state (normal: 171.1±21.6 s, kinetic threshold: 179.2±20.2 s, ECAP threshold: 237±69.9 s).

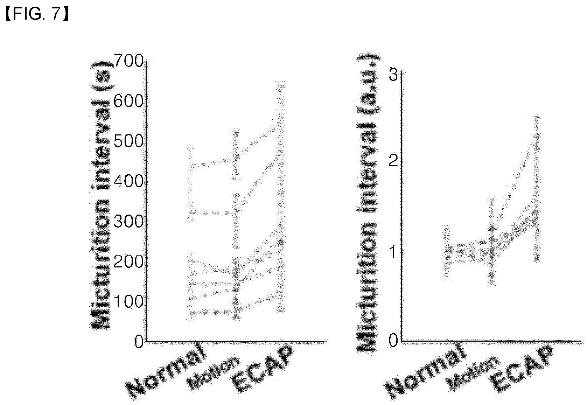

FIG. 7 presents the results of an analyzed immature interval of steady-state, motor threshold stimulation and ECAP threshold stimulation for 8 mice. Motor threshold stimulation increased the immature interval by less than 10%, while ECAP threshold stimulation achieved a significant increase. In addition to the profound effect, an immature inhibition pattern was observed only in ECAP threshold stimulation.

In the previous study (Non-Patent Documents 1 to 6), changes in electrophysiological characteristics and functional roles according to specific types of nerve fibers were described. After the stimulation pulse, the neural response was consistently observed within the signal window, and gradually increased with increasing stimulation intensity. The significant increase in ECAP amplitude observed at the ECAP threshold appears to be due to the activation of additional nerve fibers during the propagation of activity potential. As more nerve fibers are activated at the stimulation intensity that has reached the ECAP threshold, the ECAP amplitude increases cumulatively as the activation threshold is gradually met. However, the change between activated nerve fiber types suggests that the stimulation intensity of the motor threshold may not be sufficient to achieve the desired neuromodulating effect, as the fibers required to produce the desired therapeutic outcome may not be sufficiently involved. These findings highlight the limitations of existing methods. Motor responses often do not correlate with optimal treatment outcomes, resulting in inadequate neuromodulating effects. If the initial stimulation intensity is insufficient, the intensity must be increased to reach the treatment threshold. However, the motor response lacks accuracy in this adjustment process.

On the other hand, the approach according to the present invention integrates quantitative feedback of ECAP analysis to enable more accurate adjustment, leading to more targeted neuromodulation. The invention relates to our experimental results showing that the system according to the invention achieves better alignment with the desired result than the existing method

### Device verification for operation under implant conditions

The circuitry of the custom implantable device is composed of a microcontroller unit and an electrophysiology interface so that nerve stimulation and signal measurement can be operated in an integrated manner. It is also characterized by wireless communication and wireless power transfer (WPT) that can be used under implanted conditions.

FIG. 3A is a schematic diagram of the entire configuration and case insertion of the device, FIG. 3B is a test result of a temperature increase of the device during driving, and FIG. 3C is a driving power analysis result of the device.

The apparatus according to the present invention was assembled in a customized package case containing a WPT transmission coil and a Li-Po battery (FIG. 3a). This case is designed to allow both hermetic sealing and radio frequency signal penetration. Wireless control, data transmission, and WPT were successfully tested in vivo prior to transplantation experiments.

FIG. 3B shows the results of temperature tests performed during maximum amplitude stimulation and WPT charging. The temperature increased by 0.7°C. after 30 minutes and 1.2°C. after 1 hour. In the idle condition, the temperature increased by 0.6°C after 6 hours. The temperature during implantation surgery increased by 1.1°C according to the protocol and was maintained below the safety limit of 2°C of the implantable medical device.

Power consumption is mainly due to the operation of electrophysiology interfaces. Power consumption during full operation (stimulation pulse signal generation at maximum amplitude) was measured as 86.8mW, while idle state consumption was 48.5mW (FIG. 3C). The system was fully operational for 5 hours and was able to remain idle for 2 days without recharging. During WPT charging, the system worked for more than 12 hours because the TNS protocol required stimulation only for 30 minutes, so charging was unnecessary during operation.

### System evaluation of implanted free-moving rat

Customized devices and electrodes were carefully designed to meet the requirements for a fully implantable system. This process included a process of optimizing both functional and physical properties to achieve effective neuromodulation while ensuring stable performance in the in vivo environment. The verification process included rigorous testing and improvements to ensure that the device and electrode can withstand the implanted condition, ensuring that the selected TNS application can be successfully implemented.

The system was assembled according to the procedure outlined in the Methods section and transplanted into a free-moving rat.

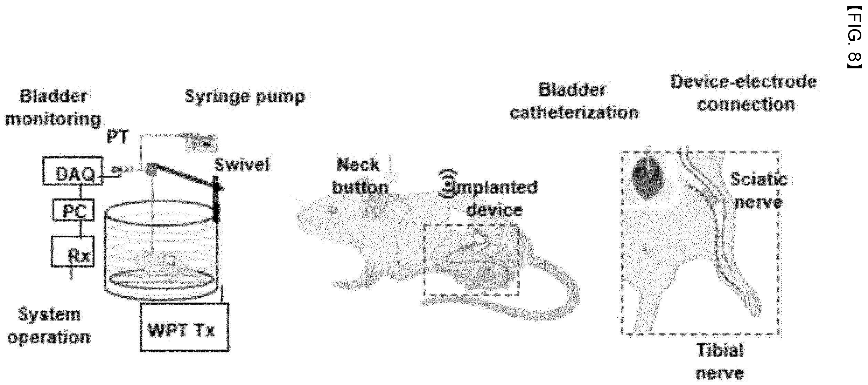

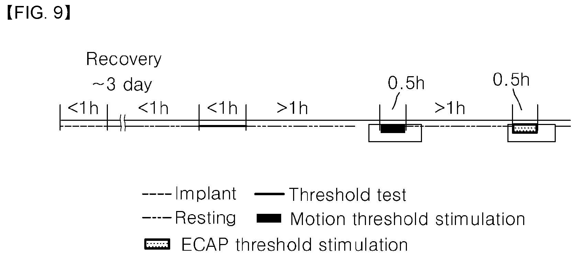

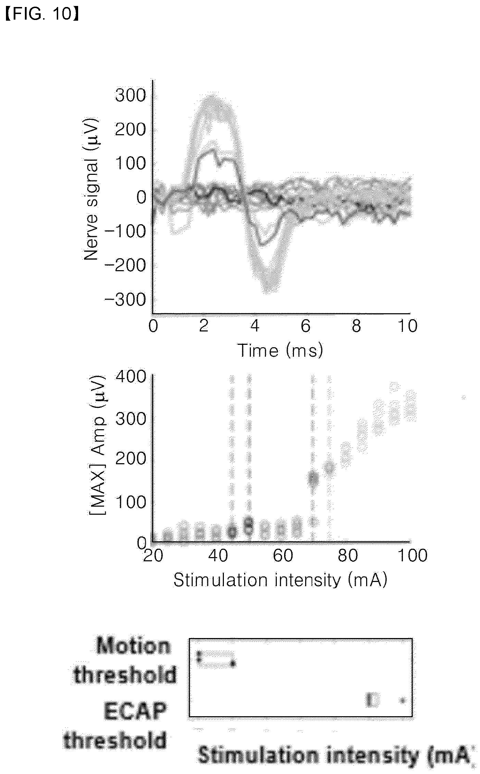

FIG. 8 is a diagram illustrating an experimental design for system testing after transplantation in an autonomic rat, and FIG. 9 is a diagram illustrating an experimental protocol timeline for experimenting in an autonomic rat. In addition, FIG. 9 is a diagram showing a threshold value analysis process performed in a rat that is performing an autonomous action (see FIG. 4), and FIG. 10 is a result of analyzing a urination pattern change as each threshold value stimulation is performed.

In the present experimental example, after a recovery period of 3 days after implantation, the rat was subjected to the experimental protocol summarized in FIG. 8 to evaluate in a free-moving state (FIG. 9). The neuronal signals measured in the transplanted conditions were successfully distinguished from the ECAP threshold (FIG. 9).

FIG. 10 shows the threshold value test and ablation interval comparison results of free-moving rat. As a result, it was found that the motion threshold was low (exercise threshold: 48±2.7 µA, ECAP threshold: 71±2.2 µA) and the effect was greater when stimulating the ECAP threshold (ECAP ablation interval change: 140.9±13.9 sec→235.0±64.7 sec).

These results, which closely match the results observed in previous acute tests, show the system's robustness and adaptability under dynamic conditions. The successful discrimination of the ECAP threshold and the consistent effect of the free-moving rat strongly indicate the reliability of the system in a more natural and real-world environment. This suggests that the effective performance of fully implanted systems can be applied to other PNS applications, which emphasizes the scalability of our approach.

As described above, the present invention is for controlling an electrical stimulation protocol in neuromodulation and is intended to solve the problems of the prior art based on sensory feeding or motor response, which is a direct response to stimulation, or waiting until a desired response is shown after stimulation. That is, unlike the related art having a problem in that it takes a long time for an effect in which a reaction occurs only after a certain time of stimulation, and in the case of motion reflection, it is not certain that the corresponding electrical stimulation induces a desired neuromodulation effect beyond motion reflection, the degree of electrical stimulation actually applied to the tibial nerve and the nervous system connected to the tibial nerve is examined through the induced complex potential, and a suitable range in which the electrical stimulation is applied is found to perform the control.

As a result, in the case of tibial nerve stimulation, a new method shows a higher neuromodulation effect compared to a conventional method of controlling through motion reflection, and for example, in a stimulation system for the purpose of overactive bladder (OAB) treatment, a stimulus-induced complex potential-based peripheral nerve stimulation based on stimulus-evoked compound action potentials (ECAPs) is fed back to monitor an appropriate stimulation intensity and treatment effect.

The present invention also provides a control method using the above-described system.

FIG. 11 is a flowchart illustrating an ECAP-based control method according to an embodiment of the present disclosure.

Referring to FIG. 11, a peripheral nerve stimulation control method according to an embodiment of the present disclosure includes: electrically stimulating a peripheral nerve through an electrode or the like; measuring an evoked compound action potential (ECAP) from the peripheral nerve generated after the electrical stimulation; determining a threshold value of the evoked compound action potential (ECAP) by receiving feedback of the measured evoked compound action potential (ECAP); and determining a stimulation intensity for stimulating the peripheral nerve according to the threshold value.

In the exemplary embodiment of the present invention, in the step of determining the threshold value of the evoked compound action potential (ECAP), the measured evoked compound action potential (ECAP) is integrated, and the threshold value becomes a point at which the second derivative of the integrated evoked compound action potential (ECAP) value exceeds two times the previous result value and shows a significant increase.

As described above, the present invention can optimize stimulation parameters using ECAP as quantitative nerve feedback, unlike the prior art having the problem that it is not certain that electrical stimulation induces a desired neuromodulation effect in addition to motor reflex.

## Claims

1. A peripheral nerve stimulation control system, the system comprising: an implantable device configured to electrically stimulate a peripheral nerve and measure evoked compound action potential (ECAP) from the peripheral nerve;
a controller configured to receive feedback of the evoked compound action potential (ECAP) measured after stimulation of the peripheral nerve and determine a threshold value of the evoked compound action potential (ECAP); and
a stimulation determiner configured to determine a stimulation intensity for stimulating the peripheral nerve according to the threshold value.

2. The peripheral nerve stimulation control system of claim 1, wherein the peripheral nerve stimulation system is implantable in vivo.

3. The peripheral nerve stimulation system of claim 1, wherein the peripheral nerve stimulation control system further comprises a wireless charging unit configured to wirelessly supply power to the device.

4. The peripheral nerve stimulation control system of claim 1, wherein the controller integrates the measured evoked compound action potential (ECAP) value.

5. The peripheral nerve stimulation control system according to claim 4, wherein the control unit determines a point at which the second derivative of the integrated evoked compound action potential (ECAP) value exceeds twice the previous result value and shows a significant increase as the threshold value.

6. The peripheral nerve stimulation control system according to any one of claim 1, wherein the peripheral nerve is a percutaneous tibial nerve.

7. A peripheral nerve stimulation control system for overactive bladder (OAB) treatment, comprising the peripheral nerve stimulation system according to claim 6.

8. A peripheral nerve stimulation control method, the method comprising:
electrically stimulating a peripheral nerve;
measuring an evoked compound action potential (ECAP) from the peripheral nerve;
receiving feedback of the measured evoked compound action potential (ECAP) and determining a threshold value of the evoked compound action potential (ECAP); and
determining a stimulation intensity for stimulating the peripheral nerve according to the threshold value.

9. The peripheral nerve stimulation control method of claim 8, wherein the determining of the threshold value of the evoked compound action potential (ECAP) comprises integrating the measured evoked compound action potential (ECAP), and the threshold value is a point at which a second derivative of the integrated evoked compound action potential (ECAP) value exceeds twice a previous result value and shows a significant increase.

10. The peripheral nerve stimulation control method of claim 9, wherein the peripheral nerve is a percutaneous tibial nerve.

11. The peripheral nerve stimulation control method of claim 9, wherein the determined stimulation strength is an electrical stimulation strength obtained by stimulating the peripheral nerve at a point where the threshold value is determined.
